# EUROPEAN PATENT APPLICATION

(11) **EP 3 085 341 A1**
(43) Date of publication of application: **26.10.2016**
(21) Application number: 16153659.4
(22) Date of filing: 31.10.2006
(51) Int. Cl.: A61F 5/00

(54) **INTRAGASTRIC SPACE FILLER**

(30) Priority: 31.10.2005 US 263302; 31.10.2005 US 262614; 22.12.2005 US 315925
(62) Divisional of application: 06827093.3
(71) Applicant: ReShape Medical, Inc., San Clemente, CA 92672 (US)
(72) Inventor: CRAGG, Andrew, Edina, Minnesota 55424 (US); QUIJANO, Rodolfo, Laguna Hills, California 92653 (US); PURKAIT, Bobby, Santa Barbara, California 93108 (US); TU, Hosheng, Newport Beach, California 92657 (US)
(74) Representative: Stephen, Robert John

(57) **Abstract**

A gastric space filler device for treating obesity in a patient by reducing the stomach volume comprising an inflatable space filler and safety element secured to the space filler, wherein the safety element yields a noticeable signal for causing removal of the space filler.

## Description

### FIELD

The present disclosure is generally related to implantable weight control devices. More particularly, the present disclosure is related to an intragastric space filler device which is retrievably implantable in a patient, owning to improved docking, stabilization, and related inherent benefits of the construction of the instant system.

### BACKGROUND OF THE PRESENT DISCLOSURE

Gastric space fillers used for achieving loss of weight in extremely obese persons have been known in the prior art. All gastric space fillers utilized for this purpose function on the principle that an empty bag or space filler is placed into the stomach through the esophagus. Thereafter, the bag or space filler is filled (fully or partially) with a suitable insufflations fluid, such as saline solution, through a filler tube or catheter which is inserted into the stomach through the mouth or the nose. The space filler occupies space in the stomach thereby leaving less room available for food and creating a feeling of satiety for the obese person. Clinical experience of the prior art has shown that for many obese patients the intragastric space fillers significantly help to control appetite and accomplish weight loss. Among the intragastric bags or space fillers described in the prior art, one type remains connected to a filler tube during the entire time period while the space filler is in the stomach. The tube is introduced into the patient's stomach through the nostrils. Such an intragastric space filler is described, for example, in U.S. Pat. No. 4,133,315.

Garren et al. in U.S. Pat. No. 4,416,267 and 4,899,747 discloses a stomach insert for treating obesity in humans by reducing the stomach volume comprising a flexible torus-shaped inflatable space filler having a central opening extending there through. At least a portion of the space filler has a self-sealing substance to facilitate puncture thereof with a needle for inflating the space filler and sealing off the puncture upon removal of the needle. The method herein comprises positioning the space filler inside the stomach of the person being treated for obesity so as to reduce the stomach volume. The Garren et al. stomach insert works satisfactorily to control the appetite. However, the insert may deflate and collapse unexpectedly resulting in obstructing the pylorus or small intestines. It appears desirable to have a space filler system that yields some noticeable warning and prompts timely removal of the implant from the patient.

Several surgical techniques have been tried which bypass the absorptive surface of the small intestine or aim at reducing the stomach size by either partition or bypass. These procedures have been proven both hazardous to perform in morbidly obese patients and have been fraught with numerous life-threatening postoperative complications. Moreover, such operative procedures are often difficult to reverse.

Non-surgical approaches for the treatment of obesity include voluntary dieting which is often unsuccessful since most persons do not possess sufficient willpower to limit the intake of food. Other approaches include the use of stomach fillers such as methylcellulose, often taken in the form of tablets. The methylcellulose expands in the stomach leaving the person with a filled-up feeling. Also, inflatable bag and tube combinations have been proposed wherein the bag is swallowed into the stomach and the tube attached thereto is used to periodically inflate the bag, particularly just prior to mealtime or during the meal. Once the person has eaten, the bag can be deflated all at once, or it can be deflated gradually over a period of a few hours so as to simulate the condition of digestion occurring and the gradual reduction of stomach contents.

U.S. Pat. No. 4,133,315 issued on January 9, 1979 discloses an inflatable bag and tube combination. The tubing remains attached to the bag and inside the esophagus of the person being treated. These tubes are often the cause of erosions and ulcerations of the esophagus. This patent also discloses a gastronomy method wherein the permanently attached tube used to distend the stomach bag extends through an opening in the stomach wall as well as an opening in the abdomen.

U.S. Pat. No. 4,246,893 issued on January 27, 1981 discloses an inflatable bag and tube combination which is surgically positioned outside and adjacent to the stomach. Upon inflation of the bag, the upper abdomen is distended and the stomach compressed to thereby produce a sense of satiety which reduces the person's desire to ingest food.

U.S. Pat. No. 4,598,699 issued on July 8, 1996 entire contents of which discloses an endoscopic instrument for removing an inflated insert from the stomach cavity of a person being treated for obesity comprising an elongated flexible tube having passageways therein and a holding device at the distal end of the flexible tube that is constructed and arranged to grasp and stabilize the inflated stomach insert.

Certain prior art discloses a gastric stimulator apparatus for stimulating neuromuscular tissue in the stomach, for example, U.S. Pat. No. 6,826,428. In one disclosure, a method of regulating gastrointestinal action using a stimulatory electrode is provided along with a sensor to provide retrograde feedback control of electrical stimulation to the GI tract or to the stomach.

U.S. Pat. No. 4,694,827 issued on September 22, 1987 discloses a balloon insertable and inflatable in the stomach to deter ingestion of food and having, when inflated, a plurality of smooth-surfaced convex protrusions disposed to permit engagement of the stomach wall by the balloon only at spaced localities, for minimizing mechanical trauma of the stomach wall by the balloon.

U.S. Pat. No. 6,746,460 issued on June 8, 2004 discloses an expandable device that is inserted into the stomach of the patient that is maintained within by anchoring or otherwise fixing the expandable device to the stomach walls. Such expandable devices have tethering regions for attachment to the one or more fasteners which can be configured to extend at least partially through one or several folds of the patient's stomach wall. Such fasteners can be formed in a variety of configurations, e.g., helical, elongate, ring, clamp, and they can be configured to be non-piercing.

Hence, reducing the size of the gastric compartment has been shown to induce weight loss in a significant percentage of people, and the present disclosure is aimed at a device which non-operatively reduces the size of the gastric compartment and which is easily removed. One aspect of the present disclosure discloses a gastric space filler device with programmed volume, adjustable capability or warning signals for device potential failure.

### SUMMARY OF THE PRESENT DISCLOSURE

According to features of the present disclosure, a gastric space filler device effective for reducing the stomach volume is disclosed comprising an inflatable space filler and a safety element secured to said space filler, wherein the safety element comprises a mechanism to yield a noticeable signal enabling removal of said space filler.

In accordance with preferred embodiments of the present disclosure, some aspects of the present disclosure relate to a gastric space filler system for treating obesity in a patient by reducing the stomach volume comprising at least two flexible inflatable space fillers secured to each other, a first space filler being inflatable to a volume inside the stomach and not in fluid communication with the other remaining space fillers, wherein at least a portion of the first-space filler is made of a biodegradable material. In an embodiment, the gastric space filler device of the present disclosure is characterized with little or minimal effects of bowel obstruction, erosion, perforation, and infection to a patient. In one preferred embodiment, the space filler generally approximates the shape of the stomach and accomplishes more complete space filling (up to 95% of stomach volume).

According to an embodiment of the present disclosure, the space filler system comprises pressure sensor element for transmitting internal pressure readings of one space filler to a receiver or controller. In a further embodiment, a pressure sensor element is mounted on a first of the at least two space fillers of the gastric space filler system for sensing an internal pressure of the first space filler. In a further embodiment, the pressure sensor element further comprises a transmitter for wirelessly transmitting the measured internal pressure signal to a receiver outside a body of the patient. The measured internal pressure is compared to a pre-determined threshold pressure for signaling removal of the filler system. In embodiments, the pressure sensor element may be substituted by a pH sensor, a flow-rate sensor, a temperature sensor, an electrolyte sensor, or the like.

In embodiments, two of the at least two space fillers of the gastric space filler system are configured to be in tandem inside the stomach pouch or are configured to be substantially parallel to each other.

According to another embodiment of the present disclosure, at least one of the two space fillers of the gastric space filler system is anchored to an inner wall of the stomach. In a further embodiment, the anchoring action is arranged and configured to activate the anchoring mechanism when the space filler is inflated while contacting the inner wall of the stomach, and to reverse the anchoring mechanism when the filler is deflated.

According to embodiments of the present disclosure, at least a portion of the at least two space fillers is ultrasonically visible. One method of visualization is to have ultrasonically visible air bubble at or on part of the space filler. Another method is to incorporate ultrasonically visible contrast agent at or on part of the space filler.

In an embodiment, the gastric space filler device is configured to be deliverable through an esophagus of the patient. In another embodiment, at least a portion of an external surface of the space filler is treated with an anti-acid substance, corrosion-resistant substance, or anti-adhesion substance, wherein the substance comprises polytetrafluoroethylene, inert material, or another biocompatible biological material (such as albumin, melatonin, phosphorylcholine, or protein). Methods of treating the surface include coating, painting, dipping, impregnation, and the like.

Aspects of the present disclosure provide a gastric space filler device for treating obesity in a patient by reducing the stomach volume comprising an inflatable space filler and a safety element secured to the space filler, wherein the safety element comprises a mechanism to yield a noticeable signal for causing a removal of the space filler.

Aspects of the present disclosure provide a gastric space filler device for treating obesity in a patient by reducing a stomach volume comprising an inflatable space filler with a first reference shape at an inflated state and means for substantially maintaining the first reference shape at a deflated state. In an embodiment, the means for substantially maintaining the first reference shape at the deflated state is to provide a spiral supportive ridgeline onto the space filler, wherein the spiral ridgeline may comprise a material similar to material of the space filler. In another embodiment, the means for substantially maintaining the first reference shape at the deflated state is to provide a plurality of cross bars inside an interior space of the splice filler.

Some aspects of the present disclosure provide a gastric space filler device for treating obesity in a patient by reducing a stomach volume comprising an inflatable space filler with a first cross-sectional circumference dimension at an inflated state and means for maintaining a second cross-sectional circumference dimension with at least 75 percent of the first circumference dimension at a deflated state.

In an embodiment, any of the at least two space fillers of the gastric space filler system have a central opening extending there through. In another embodiment, one of the at least two space fillers is fabricated from polyurethane sheet material. In still another embodiment, the polyurethane sheet material comprises a single layer. Other polymer sheet material, compliant (for example, silicone or Nylon) or non-compliant (for example, polyethylene or polytetrafluoroethylene), may also be suitable for the intended purposes.

In an embodiment, the biodegradable material for the gastric space filler system is selected from a group consisting of polymers or copolymers of lactide, glycolide, caprolactone, polydioxanone, trimethylene carbonate, polyorthoesters, and polyethylene oxide. In another embodiment, one of the at least two space fillers is made of a non-biodegradable material selected from a group consisting of polyester, polypropylene, Nylon, polyethylene, co-polymers thereof, and the like.

Aspects of the present disclosure provide a method of treating obesity in a patient with minimal nausea effects comprising implanting a stomach space filler device coated with an anti-nausea agent.

According to a feature of the present disclosure, a gastric space filler device effective for reducing the stomach volume is disclosed comprising an inflatable space filler and a safety element secured to said space filler, wherein the safety element comprises a mechanism to yield a noticeable signal enabling removal of said space filler.

### BRIEF DESCRIPTION OF THE DRAWINGS

Additional objects and features of the present disclosure will become more apparent and the invention itself will be best understood from the following Detailed Description of Exemplary Embodiments, when read with reference to the accompanying drawings.
FIG. 1 shows an embodiment of a gastric space filler device with two space fillers secured to and in parallel to each other.
FIG. 2 shows an embodiment of a first space filler of the two space fillers in FIG. 1 with a central passageway there through.
FIG. 3 shows an embodiment of a gastric space filler device with two space fillers secured to and in tandem to each other.
FIG. 4 shows an embodiment of a gastric space filler device having an inflatable space filler with a support mechanism thereto.
FIG. 5 shows an embodiment of a cross-sectional view of the support mechanism of FIG. 4.
FIG. 6 shows an embodiment of one embodiment of a gastric space filler device with two connected expandable elements.
FIG. 7 shows an embodiment of an illustration of gastric space filler device of FIG. 6 in a recipient.
FIG. 8 shows an embodiment of a gastric space filler device comprising a shape retention mechanism.
FIG. 9 shows an embodiment of a perspective view of gastric space filler device of FIG. 8.
FIG. 10 shows an embodiment of an adjustable gastric space filler device.
FIG. 11 shows an embodiment of an adjustable gastric space filler device.
FIG. 12 shows an embodiment of a delivery apparatus for non-surgically implanting a gastric space filler device.
FIG. 13 shows an embodiment of one embodiment of removing a gastric space filler device from the patient.
FIG. 14 shows an embodiment of a pressure sensor element mounted on a space filler in accordance with the principles of the present disclosure.

### DETAILED DESCRIPTION OF EXEMPLARY EMBODIMENTS

Embodiments of the present disclosure described below relate particularly to an intragastric space filler device comprising at least one space filler for reducing the stomach volume and one space filler made of biodegradable material, wherein the biodegradable material (that is, at least a portion of the space filler is made of biodegradable material) is used as a warning signal for timely removal of gastric space filler device. While the description sets forth specific details of various embodiments, it will be appreciated that the description is illustrative only and should not be construed to limit the present disclosure. Furthermore, various applications of the present disclosure, and modifications thereto, which may occur to those who are skilled in the art, are also encompassed by the general concepts described below.

The stomach is a J-shaped organ with very active muscles. Muscles of the stomach expand and contract depending on how much food is in the stomach. This contraction mechanically breaks down the food. The purpose of this breakdown is to increase the available surface area for the chemicals to act on it. The gastric glands of the stomach secrete enzymes that perform chemical breakdown, partly digesting the proteins. Pepsin is the enzyme that breakdowns protein. The gastric gland also secretes hydrochloric acid that kills almost all the bacteria in the food. It also secretes mucus that protects the stomach wall from the hydrochloric acid. By the time all the food is mechanically and chemically broken down, the food becomes a semi-fluid substance that leaves the stomach by peristalsis entering the small intestine.

The structure of the stomach is quite unique. It can be divided into four subdivisions: the cardia, the fundus, the body, and the pylorus. The cardia is the region that is closest to the heart and is where the esophagus connects to the stomach. The fundus is the region that curves above the rest of the stomach (with respects to a standing person). The body of the stomach is the central region and comprises the majority of the organ. The pylorus is the region that is connected to the small intestine. The cardia and the pylorus each have sphincter muscles that regulate the movement of food and fluids.

The volume of the human stomach varies depending on the person. Generally, human stomachs have a volume about one liter. Because the stomach has the ability to expand, however, it can hold up to four liters.

The device of the present disclosure intends to provide mechanisms for preventing or avoiding migration, bowel obstruction, bleeding diathesis, erosion, perforation of stomach or any internal organs, and the like. Some complications are acceptable if the benefits of device design far outweigh the risks, such as access site related minor complications, some patient discomfort due to the presence of the device or due to access site related issues, nausea, feeling of bloating, and the like.

Gastric space filler device is capable of filling up to 95% of stomach, and is self-adjustable or portable. It may be dialed or programmed to adjust the space filler according to input signals of pressure, volume, pH, temperature, size, electrolyte properties, etc. In an embodiment, gastric space filler device is also equipped with failure detection mechanism, such as bleeding/ulceration detection, migration limiter, etc. According to embodiments, adjustable gastric space filler device is retrievable. The device may be designed and arranged for restrictive food intake with custom shape that either adapts to or is made to the shape and size of a given patient's stomach.

Some aspects of the present disclosure provide a method for determining fluid pressure within a patient or within a space filler comprising: (a) providing a wireless capacitive MEMS chip sensor comprising an inductance coil and spaced apart capacitor plates as an inductive-capacitive circuit, with the fluid in pressure contact with one of the capacitive plates; (b) inducing a mutual inductance as an external signal into the sensor to produce the resonant frequency response as an internal signal from the sensor; and (c) determining the fluid pressure within the patient externally of the patient from the internal signal as a function of the resonant frequency response from the sensor resulting from a change in capacitance of the sensor due to a variation in the spacing of the plates produced by the fluid pressure of the fluid from the sensor resulting from the change in the series resistance. A pressure sensor element and methods of use are well known to one skilled in the art, for example the MEMS unit disclosed in U.S. Pat. No. 6,890,300 or U.S. Pat. No. 6,939,299.

Aspects of the present disclosure provide a gastric space filler device for treating obesity in a patient by reducing the stomach volume comprising an inflatable space filler and a safety element secured to the space filler, wherein the safety element yields a noticeable signal for causing a removal of the space filler, wherein at least a portion of an external surface of gastric space filler device is treated with melatonin.

Some aspects of the present disclosure provide a pressure sensor element to be mounted on a first of the at least two space fillers of gastric space filler device for sensing an internal pressure of the first space filler. In an embodiment, the pressure sensor element is mounted on the biodegradable space filler of gastric space filler device 19. In another embodiment, a pressure sensor element is mounted on any or all of the at least two space fillers of the present disclosure. In a further embodiment, the pressure sensor element further comprises a transmitter for wirelessly transmitting the measured internal pressure to a receiver outside a body of the patient or recipient.

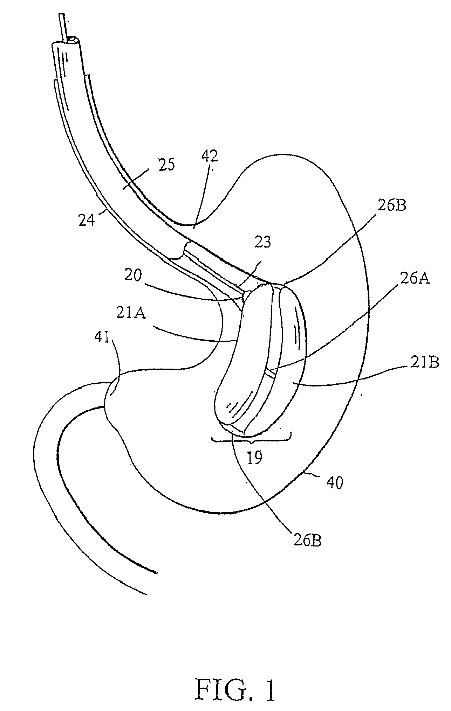

FIGS. 1-14 show embodiments of a gastric space filler device 19 and delivery tool for implanting and retrieving gastric space filler device 19 of the present disclosure. FIG. 1 shows a stomach bubble or gastric space filler device 19 with two space fillers 21A, 21B secured to and in parallel to each other.

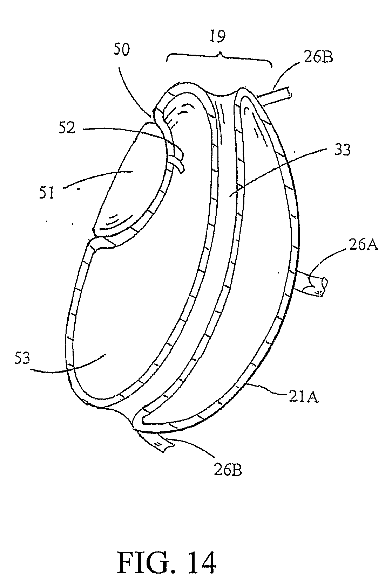

In an embodiment, gastric space filler device 19 comprises two space fillers 21 A, 21 B, wherein second space filler 21 B is enclosed within first space filler 21A, wherein at least a portion of first space filler 21A is made of a biodegradable material, optionally with a pressure sensor element (see, for example, FIG. 14) for measuring the pressure of first space filler 21A. In a further embodiment, gastric space filler device 19 comprises two space fillers 21A, 21 B, wherein second space filler 21 B is enclosed within first space filler 21A, wherein at least a portion of first space filler 21A is made of a biodegradable material with a sensor element for measuring one or more properties of the contents of the first space filler, wherein the properties include pH, temperature, electrolyte type, electrolyte concentration, and the like. In another embodiment, the space between first space filler 21A and second space filler 21 B is filled with a fluid or saline plus a dye or odor-producing mechanism for early detection that first space filler 21A is compromised, deflated, or has leaked.

In an embodiment, connecting members 26A, 26B are made of flexible or elastic material. In another embodiment, connecting members 26A, 26B are made of solid material that allows no fluid communication between space fillers 21A, 21 B. According to an embodiment, a plunger is used to push gastric space filler device 19 out of the lumen of the delivery device. In an alternate embodiment, the plunger of the delivery mechanism comprises a forward-pulling mechanism at the end distal to gastric space filler device 19. During the delivery phase, gastric space filler device 19 is under axial tension (i.e., under some pulling force between the distal end and the proximal end of the flexible space filler) to cause minimal circumferential profile for easy insertion into the sheath.

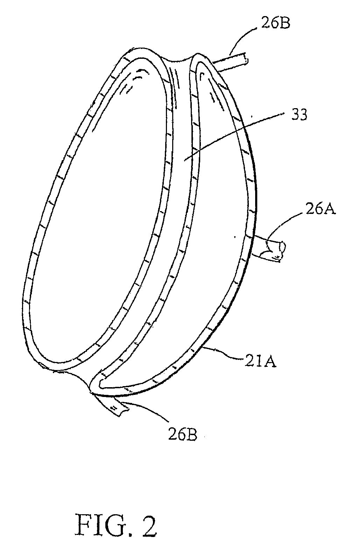

In aspects of the present disclosure, a plurality of connecting members 26A, 26B are disposed between second space filler 21 B and first space filler 21A that is connected to infusing tube 23 via sealed inlet 20. FIG. 2 shows an embodiment of first space filler 21A of FIG. 1 with a central passageway 33 there through. In an embodiment, gastric space filler device 19 comprises a plurality of passageways there through, wherein some passageways are connected to one another. In an embodiment, at least one connecting member 26A comprises a one-way check valve or seal enabling the fluid to flow from first space filler 21A into second space filler 21B, but preventing fluid from returning back to first space filler 21 A.

In an embodiment, gastric space filler device 19 is fabricated from polyurethane sheet material, wherein the polyurethane sheet material comprises a single layer. In another embodiment, gastric space filler device 19 is made of a non-biodegradable material selected from polyester, polypropylene, Nylon, polyethylene, silicone, latex, polyethylene, and copolymers thereof. In an embodiment, gastric space filler device 19 is a permanent implant. In another embodiment, gastric space filler device 19 of the present disclosure has a useful life of about 3 to 12 months.

According to still another embodiment, gastric space filler device 19 is designed for safety using an inflatable balloon made of a biodegradable material, wherein the biodegradable material is selected from a group consisting of polymers or copolymers of lactide, glycolide, caprolactone, polydioxanone, trimethylene carbonate, polyorthoesters, and polyethylene oxide. Alternately, the biodegradable material is collagen, chitosan, elastin, gelatin, and combinations thereof.

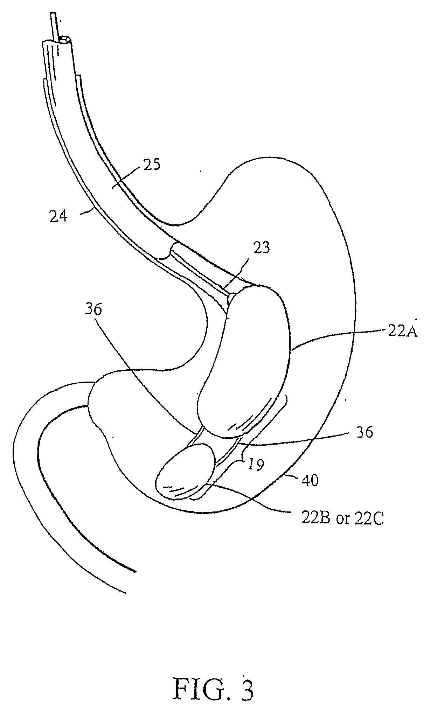

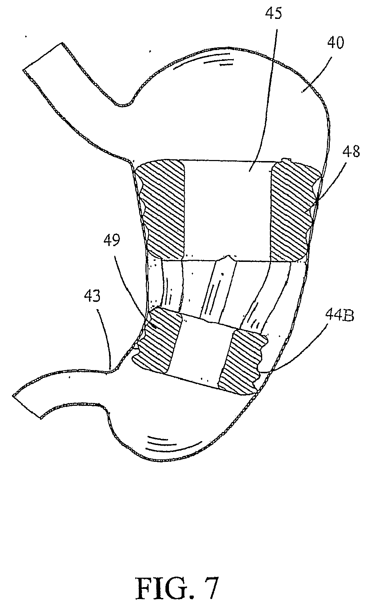

FIG. 3 shows gastric space filler device 19 with two space fillers 22A, 22B secured to and in tandem to each other. Both space fillers 22A, 22B of gastric space filler device 19 are deflated, collapsed, and retracted within catheter sheath 25 during the delivery phase or the retrieval phase of the device (see FIG. 7, for example). Gastric space filler device 19 comprises plurality of connecting members 36 between second space filler 22B and the first space filler 22A, wherein first space filler 22A is connected to infusing tube 23 via a sealed inlet.

According to embodiments of the present disclosure provide gastric space filler device 19 for treating obesity in a patient by reducing stomach volume comprising at least two flexible inflatable space fillers 22A, 22B secured to each other, each space filler 22A, 22B being inflatable to a volume inside stomach 40 and first space filler 22A is in one-way fluid communication with remaining other space fillers 22B, wherein at least a portion of the first space filler 22A is made of a biodegradable material. In an embodiment, a check valve permits the flow of fluid in only one direction from first space filler 22A through a conduit to second space filler 22B.

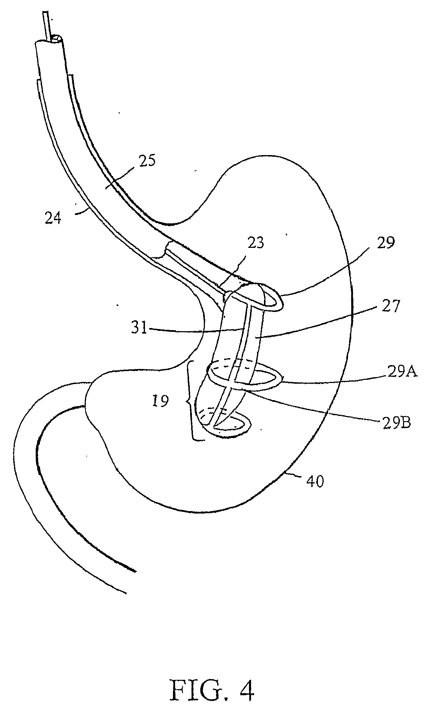

According to an embodiment shown in FIG. 4, gastric space filler device 19 is shown with an embodiment of inflatable space filler 27 and plurality of radially expanded support elements 29 thereon, wherein each support element 29, comprises suspended space filler non-contact portion 29A and space filler contact portion 29B. Radially expanded support elements 29 may be secured to each other via crossing bar 31 or other connecting mechanisms. In an embodiment, radially expanded support elements 29 are sized and configured to stabilize gastric space filler device 19 inside stomach 40 by distension against the stomach wall.

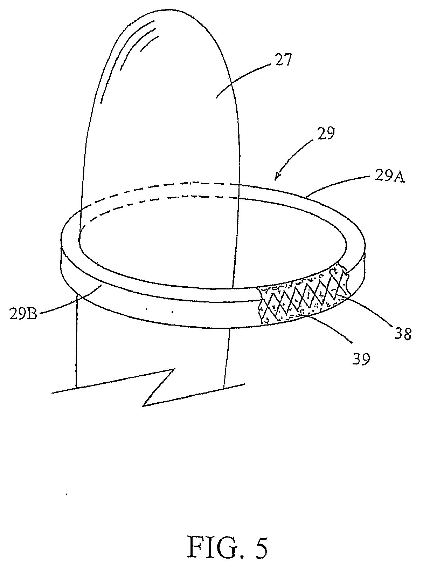

FIG. 5 shows a cross-sectional view of an exemplary support element 29 of FIG. 4. According to an embodiment, support element 29 comprises meshed stenting structure 38 wrapped or enclosed with biocompatible elastomeric material 39, such as silicone, polyurethane, latex, and the like. In an embodiment, the elastomeric material comprises a high percentage of voids or micropores, like a sponge or foam. In an embodiment, meshed stenting structure 38 is similar to a cardiovascular stent that is either self-expandable or balloon expandable. In an embodiment, meshed stenting structure 38 is mechanically crimpable or may be made of temperature sensitive shape memory Nitinol.

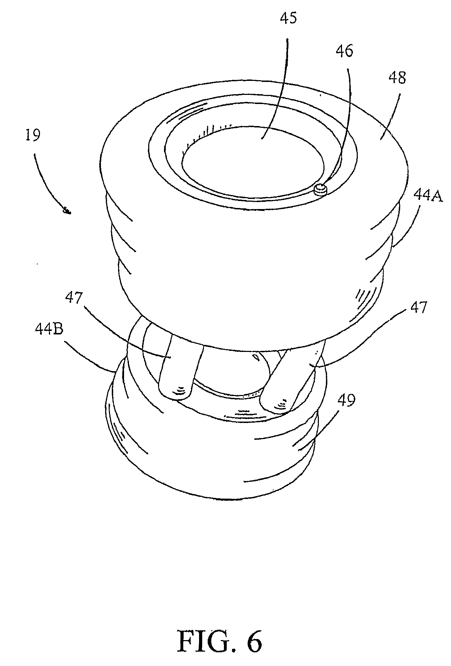

FIG. 6 shows an embodiment of gastric space filler device 19 with two connected expandable elements 48, 49. The same embodiment is shown in FIG. 7 as it would reside in stomach 40 of a patient. First expandable element 48 connects second expandable element 49 via a plurality of connecting members 47. In an embodiment, first expandable element 48 and second expandable element 49 are not in fluid communication. According to an embodiment, infusion port 46 is provided for infusion of a fluid, such as saline, or other substances into gastric space filler device 19. Infusion port 46 would be known and understood by a person of ordinary skill in the art. In another embodiment, at least one of connecting members 47 has a lumen there through for fluid communication between expandable element 48 and expandable element 49. Further, at least one of expandable elements 48, 49 has central passageway 45 for food pass-through.

According to an embodiment shown in FIGS. 6 and 7, gastric space filler device 19 is sized and configured to fit stomach volume up to 90% (effectively 95%) of the available stomach volume. In an embodiment, peripheral surface 44A of first expandable element 48 or peripheral surface 44B of second expandable element 49 has a corrugated shape so as to contact the inner wall of stomach 40 at certain discrete lines (one dimension) of the corrugation, instead of contact areas (two dimensions). In an embodiment, second expandable element 49 is sized and shaped to distend against the inner wall of stomach 40 at a place spaced away from pyloric sphincter zone 43. In an embodiment, second expanded element 49 comprises a plurality of smooth-surfaced convex protrusions disposed to permit engagement of the stomach wall by gastric space filler 19 only at spaced localities, for minimizing mechanical trauma of the stomach wall by gastric space filler 19.

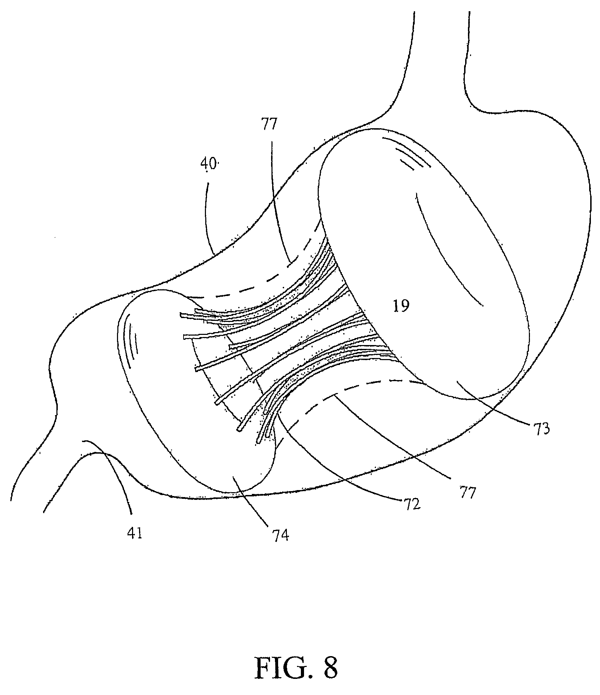

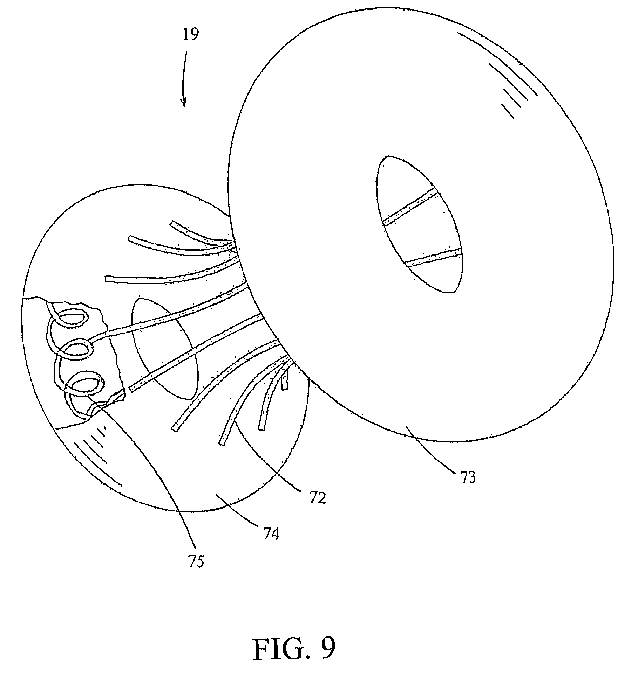

FIGS. 8 and 9 show gastric space filler device 19 with a shape retention mechanism made of different material as compared to the inflatable filler material. In an embodiment, gastric space filler device 19 comprises two toroid space fillers 73, 74. In another embodiment, first toroid space filler 73 and second toroid space filler 74 become one overall balloon-like space filler wrapped over shape retention mechanism 72 and connected by balloon-enclosed middle section 77.

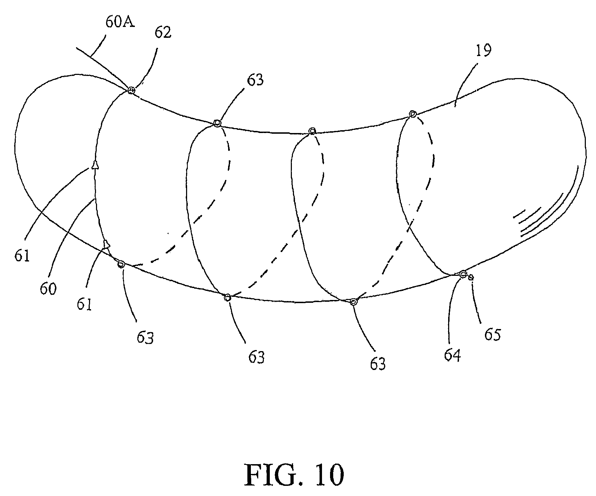

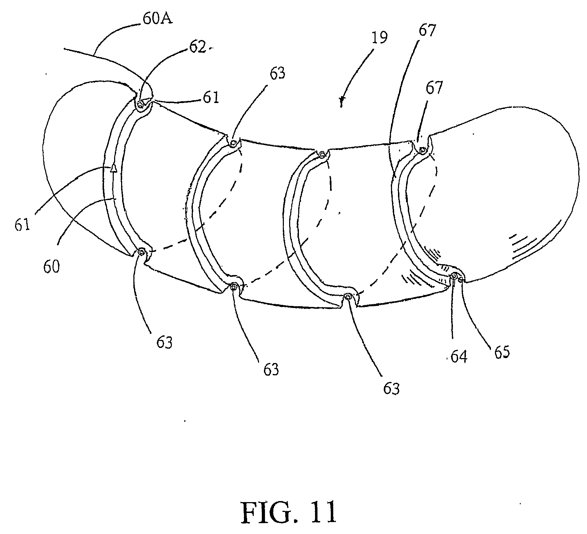

As shown in FIG. 9, shape retention mechanism 72 may further comprise spring-like coil 75 that is semi-compressible and configured to resist compressive pressure from the stomach wall, but is flexible and collapsible by a retrievable instrument either through clamping, crimping, drawing string technique (as shown in FIGS. 10 and 11), or other mechanical destructive methods as would be known and understood by artisans. Shape retention mechanism 72 may be made of Nitinol or other resilient and flexible metal or polymer.

Balloon-like space fillers are generally manufactured by dip coating a mandrel into silicone solution a few times to build up the thickness. For connecting a balloon-like space filler with another space filler or safety element, silicone compatible adhesive is generally used, for example, RTV silicone or moderate temperature curing silicone adhesive.

According to FIG. 10, there is illustrated an embodiment of gastric space filler device 19 having a mechanism of mechanically crimping the meshed stenting device 38. A person of ordinary skill in the art will know and understand that the embodiment shown in FIG. 10 is applicable to gastric space filler devices 19 having more than one space fillers. In a first step of operations, a support element is arranged and configured to be crimped circumferentially or radially inwardly to a smaller profile, together with deflated gastric space filler device 19, configured to be disposed in delivery catheter sheath 25 prior to delivery of gastric space filler device 19 in stomach 40 of a patient. In a second step of operations, the support element self-expands after releasing the constraint thereon from catheter sheath 25, along with inflated gastric space filler device 19, to occupy an appropriate space inside stomach 40. In a third step of operations during a retrieval phase, a retriever instrument with certain crimping capability is advanced into stomach 40 to orient the support element and to crimp the element to a small profile configured to be retracted within the lumen of the retriever instrument (for example, a retrieving catheter sheath). Deflated gastric space filler device 19, and together, with the crimped supporting elements, may then be withdrawn from stomach 40 to outside the body of the patient.

FIG. 10 shows an embodiment of gastric space filler device 19 whereas FIG. 11 shows another embodiment of an gastric space filler device 19. In an embodiment, gastric space filler device 19 comprises drawstring 60 coupled to the plurality of rings 62, 63, 64 that are secured to gastric space filler device 19. According to an embodiment, rings 62, 63, 64 may be an integral part of gastric space filler device 19. Distal end-knot 65 of drawstring 60 is sized larger than the opening of distal ring 64. Distal end-knot 65 keeps the distal end of drawstring 60 tight around distal ring 64. When proximal section of drawstring 60A is pulled away from gastric space filler device 19 through proximal ring 62, gastric space filler device 19 becomes smaller radially or spirally. In an embodiment, conical shaped blocker 61 can pass the ring in a one-way, ratcheting manner, as would be known to artisans. Thus, the volume of gastric space filler device 19 becomes irreversibly smaller each time conical shaped blocker 61 passes proximal ring 62. To make gastric space filler device 19 with less profile, trough 67 (FIG. 11) may be sized and configured to prevent drawstring 60 and rings 62, 63, 64 from protruding beyond the outermost external surface of gastric space filler device 19.

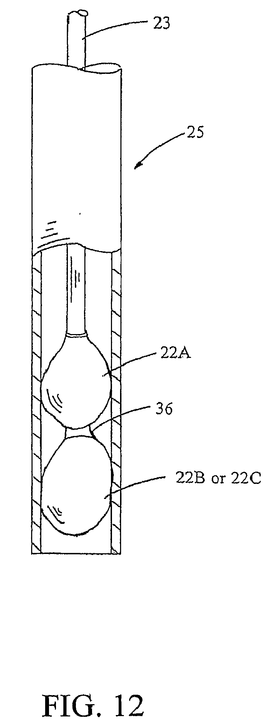

FIG. 12 shows an embodiment of standard catheter sheath 25 for deploying gastric space filler device 19 to a patient, wherein gastric space filler device 19 comprises at least two space fillers 22A and 22B, for example, connected by means of connecting members 36. Naturally, a person of ordinary skill in the art will appreciate and understand that any of space fillers disclosed herein, including 21A, 21 B, 22A, 22B, 27, 44A, 44B, 73, 74, as well as other equivalents, may be deployed from standard catheter sheath 25 in any number of embodiment configurations. In an embodiment, after advancing catheter sheath 25 into about stomach 40, infusing tube 23 serves as a pushing plunger for pushing gastric space filler device 19 into stomach 40 of the patient. Thereafter, gastric space filler devices 19 are filled with saline, fluid, or other substances depending on the embodiment, via infusing tube 23 from an external source. The apparatus of the present disclosure may then be used to treat obesity in patients by: (A) providing an inflatable gastric space filler device 19 with infusing tube 23 releasably attached thereto inside elongate catheter sheath 25, wherein gastric space filler device 19 comprises at least two flexible inflatable space fillers 22A, 22B, for example, secured to each other, along with optional safety element 22C, with first space filler 22A being inflatable to a volume inside stomach 40 wherein at least a portion of first space filler 22A is made of a biodegradable material; (B) introducing catheter sheath 25 through the mouth and into stomach 40; (C) urging gastric space filler device 19 out of catheter sheath 25 and into stomach 40; (D) inflating space fillers 22A, 22B through infusing tube 23 with a given amount of fluid, saline, or other substance to increase the volume thereof; and (E) removing infusing tube 23 from stomach 40 and out through the mouth.

Catheter sheath 25 or delivery device for gastric space filler device 19 passes through esophagus 24 and cardiac notch 42 into stomach 40 of a patient. Once it is delivered to stomach 40, space fillers 22A, 22B, for example, are inflated. In an embodiment, at least one of exemplary space fillers 22A, 22B is non-compliant and is inflated to a pressure slightly higher than the local atmospheric pressure or stomach 40 pressure, effectively to a pressure difference of about 1-20 mmHg, and more effectively to about 1-5 mmHg. One rationale of a higher pressure for exemplary space fillers 22A, 22B is to maintain the desired occupied stomach volume, though the internal pressure might fractionally drift over the course of implantation.

Phosphorylcholine (PC) is found in the inner and outer layers of cell membrane, including those found in stomach 40. It is the predominant component present in the outer membrane layer, and because it carries both a positive and negative charge (zwitterionic), it is electrically neutral. As a result, the outer layer of the cell membrane does not promote excess adhesion. When PC is coated on or incorporated on a material, protein and cell adhesion is decreased, inflammatory response is lessened, and fibrous capsule formation is minimized. Some aspects of the present disclosure relate to gastric space filler device 19 coated with an immobilized antibody (such as CD34 or the like) that mimic a biological surface for less adhesion or less reactive. It is disclosed that a method of treating obesity in a patient with minimal nausea effects comprising implanting a gastric space filler device 19 coated with an anti-nausea agent, wherein the anti-nausea agent may be melatonin, albumin, or phosphorylcholine to mimic a biological surface.

According to embodiments, at least a portion of an external surface of gastric space filler device 19 is treated with an anti-acid substance or an anti-adhesion substance. According to further embodiments, gastric space filler device 19 has a central opening extending there through or gastric space filler device 19 is sized to occupy at least 90% of stomach volume of the patient. In a further embodiment, gastric space filler device 19 has an adjustable volume and is sized and configured to occupy up to 90% of stomach volume (effectively 95%) of the patient. Gastric space filler device 19 should be able to be increased in size over time through port infusion or re-docking infusion, as would be known to artisans. The size of gastric space filler device 19 may be adjusted over time to allow initial acceptance by stomach 40 and increased volume to achieve the right balance between weight loss and the lack of nausea and vomiting.

In an embodiment, at least a portion of an external surface of gastric space filler device 19 is treated with an anti-acid substance, corrosion-resistant substance, or anti-adhesion substance, wherein the substance comprises polytetrafluoroethylene, inert material, or other biological material (such as albumin, melatonin, phosphorylcholine, immobilized antibody, or proteins) that are biocompatible. Methods of treating the surface include coating, painting, dipping, impregnation, and the like. In an embodiment, the melatonin or phosphorylcholine coating is on at least a portion of the outer surface of gastric space filler device 19. In an effective embodiment, the melatonin or phosphorylcholine coating is on at least the portion of the outer surface of gastric space filler device 19 that contacts the stomach wall. Gastric space filler device 19 may also be made of or surface coated with polyolefin family like high density polyethylene; linear low density polyethylene; and ultra-high molecular weight polyethylene; fluoropolymer materials, like fluorinated ethylene propylene, polymethylpentene, polysulphons; or some elastomers such as thermoplastic polyurethanes and C-Flex type block copolymers.

Turning back to FIG. 1, the inner surface or the outer surface of delivery catheter sheath 25 may be treated to be hydrophilic or to have reduced surface friction. Gastric space filler device 19 has sealed inlet 20 that allows fluid or saline to be infused into space fillers 21A, 21B via infusing tube 23 or a needle connected to infusing tube 23 that is connected to an external fluid source. In an embodiment, sealed inlet 20 is made of a self-sealing substance to facilitate puncture thereof with a needle for inflating space filler 21 A, 21B and sealing off the puncture upon removal of the needle. In another embodiment, sealed inlet 20 is equipped with a one-way check valve for receiving infusing fluid or saline. Artisans will understand the applicability of these principles to each of embodiments disclosed herein, as well as equivalents.

Similarly, administration of melatonin may reduce nausea associated with implantation of gastric space filler device 19. Melatonin is a sleep promoting agent that is involved in the regulation of gastrointestinal motility and sensation. In some prior clinical experiments, melatonin was orally administered 3 mg at bedtime for two weeks, those patients with melatonin regimen show significant attenuation in abdominal pain and reduced sensitivity in rectal pain as compared to the control group with placebo.

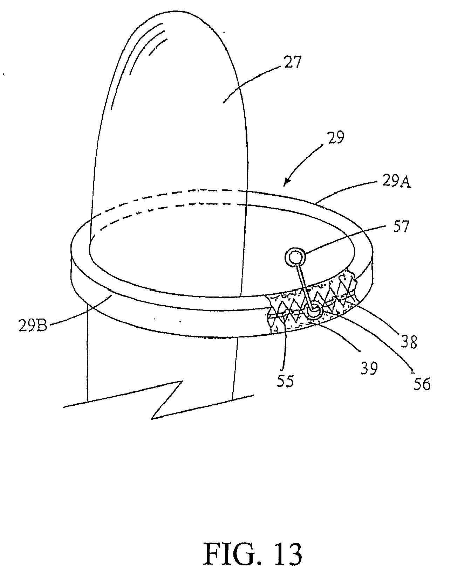

According to a similar safety/retrieval device shown in FIG. 13, at about the middle section of meshed stenting device 38 circumferentially, mesh struts cross. struts. Tether 55 extends through a front side and a backside of each crossing point alternately. Tether 55 is then joined in loop 56 with one end of the tether extending through loop 56 in the other end of the tether and extending slightly out of the plane with end loop 57. At a retrieval time, end loop 57 is snatched or grasped by a retriever apparatus (such as the apparatus having a hook, a grasper, or the like) and pulled toward outside of the mouth, enabling collapsing circumferentially the stenting structure to a much smaller profile for removal out of the body.

According to embodiments, other safety features are likewise provided. The biodegradable portion of the biodegradable space filler is sized and configured to biodegrade at a specified time duration, the biodegradation of the biodegradable space filler and its subsequent deflation serving as a warning signal for retrieving gastric space filler device 19. There is provided a safety feature when pressure sensors on space fillers emit a low-pressure signal as a result of space filler deflation. Some aspects of the present disclosure provide a signal for removal or retrieval of gastric space filler device 19 when space filler 21A, 21 B, for example, is deflated or signaled to have low pressure. This would prevent the catastrophic or life-threatening blocking/obstructing of pylorus 41 by a completely deflated gastric space filler device 19. In an embodiment, gastric space filler device 19 might be retrieved at a predetermined post-implantation time, for example at 6 months post-implantation.

Some aspects of the present disclosure provide gastric space filler device 19 for treating obesity in a patient by reducing stomach volume comprising inflatable space filler with a reference shape that is substantially maintained after gastric space filler device 19 is deflated by accident or intentionally. For illustrative purposes, maintenance of the reference shape of gastric space filler device 19 occurs due to the incorporation of a relatively rigid supportive spiral ridgeline along the interior surface of gastric space filler device 19. The supportive ridgeline is similar to the reinforcing spiral elements along an internal surface of a hose. The ridgeline is sized (at least one complete hoop circle) and configured to resist compressive pressure from the stomach wall, but is flexible and collapsible by a retrievable instrument either through clamping, crimping, drawing string technique, or other mechanical destructive methods. In an embodiment, the ridgeline is made of the same biocompatible material as gastric space filler device 19. In another embodiment, the ridgeline is an integral part of gastric space filler device 19. In still another embodiment, the ridgeline contains a wholly enclosed elastic metal wire or coil by the same biocompatible material of gastric space filler device 19.

For further illustrative purposes, the device for maintaining the reference shape of gastric space filler device 19 incorporates a plurality of relatively rigid cross bars inside the interior space of gastric space filler device 19, wherein each end of the cross bars is secured to the interior wall of gastric space filler device 19. The structure of the cross bars is sized and configured to resist compressive pressure from the stomach wall, but is flexible and collapsible by a retrievable instrument either through clamping, crimping, or other mechanical destructive methods. In an embodiment, the cross bar is made of the same biocompatible material as gastric space filler device 19. For further illustrative purposes, the device for maintaining the reference shape of gastric space filler device 19 is to incorporate a foam material inside the interior volume of gastric space filler device 19. The structure of the foam material is sized and shaped to resist compressive pressure from the stomach wall, but is flexible and collapsible by a retrievable instrument either through clamping, crimping, drawing string technique (as shown in FIGS. 10 or 11), or other mechanical methods. By maintaining the shape of gastric space filler device 19 substantially similar to the reference shape after gastric space filler device 19 is deflated by accident or intentionally would cause gastric space filler device 19 remain inside stomach 40 and not to obstruct the bowel.

FIG. 14 shows an embodiment of space filler 21A (see, e.g., FIG. 2) having pressure sensor element 51. Pressure sensor elements 51 may be suitable pressure sensors as known to a person of ordinary skill in the art, for example the pressure sensor disclosed in U.S. Pat. No. 6,890,300. Pressure sensor element 51 may be mounted on space filler 21A in accordance with the principles of the present disclosure or as known to artisans. In an embodiment, space filler 21A comprises recess area 50 sized and configured to appropriately receive and mount pressure sensor element 51. Pressure sensor element 51 is in pressure communication with internal void 53 via opening 52.

Moreover, turning again to FIG. 3, gastric space filler device 19 may comprise flexible inflatable space filler 22A and safety element 22C, which are connected to space filler 22A, wherein safety element 22C yields a noticeable signal for causing removal of gastric space filler device 19. In an embodiment, safety element 22C is a visible dye that, when gastric space filler device 19 is compromised, causes a visible change to the appearance of urine shortly after gastric space filler device 19 is breached. In an embodiment, safety element 22C comprises an odor that, when gastric space filler device 19 is compromised or leaked, causes an odor in the urine shortly after breach of gastric space filler device 19. In a further embodiment, safety element 22C comprises biodegradable material that, when safety element 22C biodegrades prematurely, yields a signal for prompt removal of the decomposed/biodegraded pieces. Implementation of safety device 22C will be understood by artisans, who will recognize the variations and combination of signaling devices alerting patients or doctors of the need to remove gastric space filler device 19.

Safety element 22C may also comprise a pressure sensor element for sensing an internal pressure of space filler 22A, as previously described. The pressure sensor element may further comprise a transmitter for wirelessly transmitting a measured internal pressure to a receiver outside a body of the patient. In an embodiment, safety element 22C comprises a pH sensor element for sensing a pH of stomach 40 of the patient, wherein the pH sensor element may further comprise a transmitter for wirelessly transmitting the sensed pH to a receiver outside a body of the patient. The sensed pH or the change of the sensed pH with respect to time is compared to the historic data or pre-determined data for assessing device performance or integrity. If the sensed pH is below the threshold number for a predetermined period, this signal may prompt retrieval of gastric space filler device 19.

Safety element 22C may comprise device for maintaining the shape of gastric space filler device 19 so the compromised gastric space filler device 19 (either via leaking or collapsing) does not cause bowel obstruction. Safety element 22C may maintain the residual cross-sectional shape or circumference dimension after a filler compromise, for example as shown in an embodiment shown in FIG. 4. In an embodiment, the appropriate dimension retention is at least 50%, to 75%, of the pre-compromised reference value.

According to an embodiment, gastric space filler device 19 and safety element 22C are configured to be in tandem inside stomach 40. In another embodiment, gastric space filler device 19 and safety element 22C are configured to be substantially parallel to each other. In a further embodiment, safety element 22C or gastric space filler device 19 containing safety element 22C is anchored to or anchored through an inner wall of stomach 40. Artisans will understand and appreciate these principles and the related implementations.

In an embodiment, safety element 22C or space filler (*e*.*g*., 21A, 21B) of gastric space filler device 19 is ultrasonically visible. In another embodiment, an ultrasonic transducer is mounted on either the safety element or gastric space filler device 19 for emitting an ultrasonic signal.

Aspects of the present disclosure relate to an anchoring or securing mechanism of gastric space filler device 19 that anchors only when gastric space filler device 19 is adequately inflated. In an embodiment, at least one of (*e*.*g*., 21A, 21B) space fillers of gastric space filler device 19 is anchored to an inner wall of stomach 40. In a further embodiment, the anchoring action is arranged and configured to activate the anchoring mechanism (such as from a piercing needle) when gastric space filler device 19 is inflated while contacting the inner wall of stomach 40, and to reverse the anchoring mechanism when the filler is deflated. Inflated gastric space filler device 19 is maintained within or stabilized by anchoring or otherwise securing the expandable device to the stomach walls. In an embodiment, such expandable devices have tethering regions for attachment to the one or more fasteners which can be configured to extend at least partially through one or several folds of the patient's stomach wall as would be known to artisans. Such fasteners can be formed in a variety of configurations, e.g., helical, elongate, ring, clamp, and they can be configured to be non-piercing. Persons of ordinary skill will understand how to anchor gastric space filler device 19 to stomach wall and the attendant issues related to anchoring.

While the apparatus and method have been described in terms of what are presently considered to be the most practical and preferred embodiments, it is to be understood that the disclosure need not be limited to the disclosed embodiments. It is intended to cover various modifications and similar arrangements included within the spirit and scope of the claims, the scope of which should be accorded the broadest interpretation so as to encompass all such modifications and similar structures. The present disclosure includes any and all embodiments of the following claims.

### STATEMENTS OF INVENTION

S1. A gastric space filler device effective for reducing the stomach volume comprising inflatable space filler and a safety element secured to said space filler, wherein the safety element comprises a mechanism to yield a noticeable signal enabling removal of said space filler.
S2. The gastric space filler device of statement S1, wherein the safety element comprises a pressure sensor element for sensing an internal pressure of said inflatable space filler and wherein the space filler and the safety element are configured to be in tandem or substantially parallel to each other inside a stomach pouch of the patient, and at least one of the space filler and safety element is anchored to an inner wall of a stomach pouch of the patient.
S3. The gastric space filler device of statement S2, wherein the pressure sensor element further comprises a transmitter for wirelessly transmitting the sensed internal pressure to a receiver outside a body of the patient.
S4. The gastric space filler device of statement S2, wherein the safety element comprises a pH sensor element for sensing a pH of a stomach of the patient, in combination with a transmitter for wirelessly transmitting the sensed pH to a receiver outside a body of the patient
S5. The gastric space filler device of statement S2, wherein the safety element or the space filler is ultrasonically visible.
S6. The gastric space filler device of statement S2, wherein an ultrasonic transducer is provided for emitting an ultrasonic signal.
S7. The gastric space filler device of statement S1, wherein the gastric space filler is configured to be deliverable through an esophagus of the patient.
S8. The gastric space filler device of statement 2, wherein at least a portion of an external surface of the space filler device is treated with at least one of an anti-acid substance, a corrosion-resistant substance, an anti-adhesion substance, with albumin or melatonin, and phosphorylcholine.
S9. The gastric space filler device of statement 2, wherein the space filler comprises a central opening extending there through.
S10. The gastric space filler device of statement 2, wherein the safety element is further comprised of an is an inflatable balloon made of at least one of a biodegradable material is selected from a group consisting of polymers or copolymers of lactide, glycolide, caprolactone, polydioxanone, trimethylene carbonate, polyorthoesters, polyethylene oxide, collagen, chitosan, elastin, gelatin, and combinations thereof.

## Claims

1. A gastric space filler device for treating obesity in a patient by reducing the patient's stomach volume, the gastric space filler device comprising:
a plurality of inflatable gastric space fillers including at least a first inflatable gastric space filler and a second inflatable gastric space filler;
at least one connecting member extending between and connected to the first and second inflatable gastric space fillers, wherein opposing ends of the first and second inflatable gastric space fillers are spaced apart from each other by the connecting member; and
a safety element configured to yield a noticeable signal for causing a removal of the gastric space filler device from the patient.

2. The gastric space filler device of claim 1, wherein the safety element is a pressure sensor element configured to sensing an internal pressure of at least one of the first inflatable gastric space filler and the second inflatable gastric space filler.

3. The gastric space filler device of claim 1 wherein the safety element includes a transmitter configured to wirelessly transmitting at least one of a sensed pressure or a sensed pH to a receiver outside a body of the patient.

4. The gastric space filler device of claim 1, wherein the safety element includes a pH sensor element for sensing a pH of a stomach of the patient.

5. The gastric space filler device of any one of claims 1-4, wherein the safety element and/or at least one of the first and/or second inflatable gastric space fillers are ultrasonically visible.

6. The gastric space filler device of any one of claims 1-5, wherein the gastric space filler device is configured to be deliverable through an esophagus of the patient.

7. The gastric space filler device of any one of claims 1-6, wherein at least a portion of an external surface of the gastric space filler device is treated with at least one of an anti-acid substance, a corrosion-resistance substance, an anti-adhesion substance, albumin, melatonin, or phosphorylcholine.

8. The gastric space filler device of any one of claims 1-7, wherein the inflatable space filler includes a central opening extending therethrough.

9. The gastric space filler device of any one of claims 1-8, wherein at least one of the first and/or second inflatable gastric space fillers has an adjustable volume.

10. The gastric space filler device of claim 1, wherein at least a portion of the first inflatable gastric space filler is made of a biodegradable material.

11. The gastric space filler device of claim 1, wherein the safety element is secured to the first and/or second inflatable gastric space fillers.

12. The gastric space filler device of claim 1, wherein the safety element is one of the first and/or second inflatable gastric space fillers.

13. The gastric space filler device of any one of claims 1-11, wherein the first inflatable gastic space filler is configured to be inflated to a first volume in a stomach of the patient and the second inflatable gastic space filler is configured to be inflated to a second volume inside the stomach of the patient, and wherein the first volume and the second volume are configured to occupy at least 90% of the stomach volume.

14. The inflatable gastric device of claim 1, wherein the safety element includes a dye.

15. The gastric space filler device of claim 1, further comprising:
an infusing tube releasably attached to the gastric space filler device, wherein the infusing tube is configured to be connected to an external fluid source to inflate the inflatable gastric space fillers when the inflatable gastric space fillers are positioned within a stomach of the patient, and wherein the infusing tube is removed from the patient after inflation.
